# EUROPEAN PATENT SPECIFICATION

(11) **EP 4 053 200 B1**
(45) Date of publication and mention of the grant of the patent: **23.07.2025**
(21) Application number: 20881611.6
(22) Date of filing: 07.09.2020
(51) Int. Cl.: C08K 5/053, C08K 5/09, C08L 83/08, A61Q 5/12, C08L 71/02, A61K 8/34, A61K 8/36, A61K 8/365, A61K 8/45, A61K 8/898, C08K 3/20, D06M 15/643, A61K 8/02, A61Q 19/00, A61K 8/06

(54) **EMULSION OF AMINO-MODIFIED SILICONE, HAIR COSMETIC, AND TEXTILE TREATMENT AGENT**
EMULSION VON AMINOMODIFIZIERTEM SILIKON, HAARKOSMETIKUM UND TEXTILBEHANDLUNGSMITTEL
ÉMULSION DE SILICONE AMINO-MODIFIÉE, AGENT DE TRAITEMENT COSMÉTIQUE CAPILLAIRE ET TEXTILE

(30) Priority: 30.10.2019 JP 2019197925
(43) Date of publication of application: 07.09.2022
(73) Proprietor: Shin-Etsu Chemical Co., Ltd., Tokyo 100-0005 (JP)
(72) Inventor: ANDO, Yuko, Annaka-shi, Gunma 379-0224 (JP)
(74) Representative: Sonnenhauser, Thomas Martin
(86) International application number: PCT/JP2020/033704
(87) International publication number: WO 2021/084912

(56) References cited:
- JP-A- 2001 226 222
- JP-A- 2002 060 331
- JP-A- 2011 001 419
- JP-A- 2013 220 991
- JP-A- 2018 172 491
- US-A- 5 057 572
- US-A- 5 712 343
- US-A1- 2014 308 229
- "New O/W microemulsion ED - Darl Kuhn", IP.COM, IP.COM INC., WEST HENRIETTA, NY, US, 4 April 2018 (2018-04-04), XP013178287, ISSN: 1533-0001

## Description

### TECHNICAL FIELD

The present invention relates to: an amino-modified silicone emulsion; a hair cosmetic; and a fiber treatment agent.

### BACKGROUND ART

Hair cosmetics such as shampoo, conditioner, and treatment are desired to have excellent conditioning effects on hair such as softness, smoothness, and an emollient effect after use.

Since gloss and smooth running of fingers through the hair become poor after washing the hair with shampoo or the like, a hair conditioner etc. is used after a shampoo. A hair conditioner usually contains a cationic surfactant, oil content, and so forth as main components.

The use of dimethyl silicone having a high polymerization degree and amino-modified silicone as oil contents in a hair cosmetic is well known. In particular, amino-modified silicone adheres to hair easily, and is excellent in softness, smoothness, and emollient effect (Patent Document 1).

It is also known that amino-modified silicone is also useful for fiber treatment agents such as detergents for washing and softeners. It is known that amino-modified silicone acts on washing in a wet state after dehydration in washing, and has an effect of providing excellent texture (Patent Document 2).

Furthermore, it is known that amino-modified silicone is also useful as an anti-contamination agent for roll contamination of a paper machine in a papermaking process, and that by providing an amino-modified silicone emulsion to a roll in a drying process or the like, contamination such as pitch can be prevented (Patent Document 3).

In this manner, amino-modified silicone emulsions are used in many fields for providing softness or for stain-proofing agents.

In recent years, regulations regarding cyclic low-molecular-weight siloxanes (octamethyl cyclotetrasiloxane, decamethyl cyclopentasiloxane, and dodecamethyl cyclohexasiloxane) have become stricter in various countries, and the reduction of cyclic low-molecular-weight siloxanes in silicones has become an issue. In particular, in products that are drained as waste water after use such as hair cosmetics including shampoos and conditioners or detergents and softeners for washing, the amount of cyclic low-molecular-weight siloxanes contained in the product is sometimes subject to regulation.

As stated above, amino-modified silicone is effective for hair cosmetics such as shampoos and conditioners and fiber treatment agents such as detergents and softeners for washing. On the other hand, however, cyclic low-molecular-weight siloxanes (such as octamethyl cyclotetrasiloxane and decamethyl cyclopentasiloxane) are produced in an amino-modified silicone emulsion as by-products during storage. Therefore, the following have been considered until now.

Patent Document 4 proposes a method for producing an emulsion using polydialkylsiloxane, aminofunctional organopolysiloxane, a quaternary ammonium surfactant, and water. In Patent Document 4, the inclusion of the quaternary ammonium surfactant is essential, and if an emulsion containing a quaternary ammonium surfactant is blended in a shampoo or the like containing an anionic surfactant, there is risk of degrading stability.

Patent Document 5 proposes a process of applying to a fiber an emulsion of aminofunctional organopolysiloxane, a quaternary ammonium surfactant, a nonionic surfactant, and water, and it is reported that octamethyl cyclotetrasiloxane (D4) and decamethyl cyclopentasiloxane (D5) in the emulsion hardly increase over time compared with conventional methods. However, the inclusion of the quaternary ammonium surfactant is essential, and as in the case of Patent Document 4, there is risk of degrading stability if an emulsion of Patent Document 5 is blended in a shampoo or the like containing an anionic surfactant.

Patent Document 6 reports an emulsion composition containing aminofunctional organopolysiloxane, a quaternary ammonium surfactant, a nonionic surfactant, and water and having less than 0.1 mass% of octamethyl cyclotetrasiloxane (D4) and decamethyl cyclopentasiloxane (D5). However, the inclusion of the quaternary ammonium surfactant is essential, and in the same manner as Patent Document 4 and Patent Document 5, there is risk of degrading stability if an emulsion of

Patent Document 6 is blended in a shampoo or the like containing an anionic surfactant.

### CITATION LIST

### PATENT LITERATURE

Patent Document 1: JP 5794637 B2
Patent Document 2: JP 2008-150756 A
Patent Document 3: JP 5960378 B1
Patent Document 4: JP 2013-532741 A
Patent Document 5: JP 2015-500926 A
Patent Document 6: JP 6258213 B2

### SUMMARY OF INVENTION

### TECHNICAL PROBLEM

The present invention has been made in view of the above-described circumstances, and an object thereof is to provide an amino-modified silicone emulsion in which octamethyl cyclotetrasiloxane (D4), decamethyl cyclopentasiloxane (D5), and dodecamethyl cyclohexasiloxane (D6) hardly increase over time compared with conventional products, and which provides excellent smoothness and softness when blended in hair cosmetics or fiber treatment agents.

### SOLUTION TO PROBLEM

To achieve the object, the present invention provides an amino-modified silicone emulsion comprising:
(A) an amino-modified silicone that is represented by the following general formula (1), has a viscosity at 25°C of 200 to 100,000 mPa·s, and has an amine equivalent of 1,000 to 20,000 g/mol, 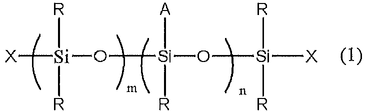 wherein R represents a substituted or unsubstituted monovalent hydrocarbon group having 1 to 12 carbon atoms, A represents a monovalent group shown by - R¹(NR²CH₂CH₂)ₐNR³R⁴, wherein R¹ represents a divalent hydrocarbon group having 1 to 8 carbon atoms, R², R³, and R⁴ each independently represent a hydrogen atom or a monovalent hydrocarbon group having 1 to 4 carbon atoms, and "a" represents an integer of 0 to 4, X represents a monovalent group shown by R, A, or -OR⁵, R⁵ represents a hydrogen atom or a monovalent hydrocarbon group having 1 to 8 carbon atoms, and "m" and "n" are mean values and are numbers that satisfy the viscosity and the amine equivalent, provided that when n=0, at least one X is A, and that octamethyl cyclotetrasiloxane (D4), decamethyl cyclopentasiloxane (D5), and dodecamethyl cyclohexasiloxane (D6) are each contained in an amount of less than 3,000 ppm relative to a mass of the component (A);
(B) 5 to 100 mass% of a nonionic surfactant relative to the mass of the component (A);
(C) 50 to 100 mol% of lactic acid or acetic acid relative to a number of moles of amino groups of the component (A); and
(D) 85 to 10,000 mass% of water relative to the mass of the component (A),
wherein an increased amount of each of (D4), (D5), and (D6) is less than 3,000 ppm relative to the mass of the component (A) when an emulsion of the amino-modified silicone has been stored at 25°C for 6 months.

In the inventive amino-modified silicone emulsion, (D4), (D5), and (D6) hardly increase over time compared with conventional products, and by blending the inventive amino-modified silicone emulsion in a hair cosmetic or a fiber treatment agent, excellent smoothness and softness can be provided.

The inventive amino-modified silicone emulsion preferably comprises (E) 1 to 20 mass% of a polyhydric alcohol relative to the mass of the component (A).

Such an amino-modified silicone emulsion has reduced viscosity and is easier to handle in some cases, and in some cases, increase in (D4), (D5), and (D6) in the amino-modified silicone emulsion over time can be suppressed. In addition, when the inventive amino-modified silicone emulsion containing the component (E) is blended in a hair cosmetic or a softener, moisturizing property can be provided.

Furthermore, the inventive amino-modified silicone emulsion may comprise (F) 0.1 to 3 mass% of an anionic surfactant relative to the mass of the component (A).

When the component (F) is blended as described, the viscosity of the amino-modified silicone emulsion is reduced, and handling becomes easier.

The nonionic surfactant (B) is preferably a polyoxyalkylene alkyl ether.

Thus, a polyoxyalkylene alkyl ether can be used suitably as the nonionic surfactant (B).

Furthermore, the increased amount of each of (D4), (D5), and (D6) is preferably less than 2,000 ppm relative to the mass of the component (A) when stored at 25°C for 6 months.

Such an amino-modified silicone emulsion has an even lower risk of having the blended amount restricted by legal regulations when the inventive amino-modified silicone emulsion is blended in a product for use in a hair cosmetic, a softener, etc., which are drained as waste water after use.

In addition, the present invention provides a hair cosmetic comprising 0.1 to 20 mass% of the above-described amino-modified silicone emulsion.

Furthermore, the present invention provides a fiber treatment agent comprising 0.1 to 20 mass% of the above-described amino-modified silicone emulsion.

In the hair cosmetic and the fiber treatment agent having the inventive amino-modified silicone emulsion blended, (D4), (D5), and (D6) hardly increase during storage, and the hair cosmetic and the fiber treatment agent can provide hair, fiber, or the like with excellent smoothness and softness.

### ADVANTAGEOUS EFFECTS OF INVENTION

As described above, the inventive amino-modified silicone emulsion contains: an amino-modified silicone having a specific amine equivalent and a specific viscosity; a nonionic surfactant; and a specific amount of lactic acid or acetic acid. In addition, the inventive amino-modified silicone emulsion has a characteristic that octamethyl cyclotetrasiloxane (D4), decamethyl cyclopentasiloxane (D5), and dodecamethyl cyclohexasiloxane (D6) hardly increase over time compared with conventional products. Moreover, by blending the amino-modified silicone emulsion in a hair cosmetic or a fiber treatment agent such as a softener, excellent smoothness and softness can be provided.

### DESCRIPTION OF EMBODIMENTS

As described above, it has been desired to develop an amino-modified silicone emulsion that can provide hair, fiber, etc. with excellent smoothness and softness, and in which cyclic low-molecular-weight siloxanes are hardly produced as by-products during storage.

To achieve the object, the present inventor has earnestly studied and found out that by emulsifying an amino-modified silicone having a specific amine equivalent and viscosity and having a specific structure with specific amounts of lactic acid or acetic acid and nonionic surfactant, it is possible to obtain an amino-modified silicone emulsion in which octamethyl cyclotetrasiloxane (D4), decamethyl cyclopentasiloxane (D5), and dodecamethyl cyclohexasiloxane (D6) hardly increase over time compared with conventional products. Furthermore, the present inventor has found out that by blending the amino-modified silicone emulsion in a hair cosmetic or a fiber treatment agent, excellent smoothness and softness can be provided, and arrived at the present invention.

That is, the present invention is an amino-modified silicone emulsion comprising:
(A) an amino-modified silicone that is represented by the following general formula (1), has a viscosity at 25°C of 200 to 100,000 mPa·s, and has an amine equivalent of 1,000 to 20,000 g/mol, 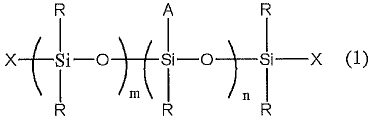 wherein R represents a substituted or unsubstituted monovalent hydrocarbon group having 1 to 12 carbon atoms, A represents a monovalent group shown by - R¹(NR²CH₂CH₂)ₐNR³R⁴, wherein R¹ represents a divalent hydrocarbon group having 1 to 8 carbon atoms, R², R³, and R⁴ each independently represent a hydrogen atom or a monovalent hydrocarbon group having 1 to 4 carbon atoms, and "a" represents an integer of 0 to 4, X represents a monovalent group shown by R, A, or -OR⁵, R⁵ represents a hydrogen atom or a monovalent hydrocarbon group having 1 to 8 carbon atoms, and "m" and "n" are mean values and are numbers that satisfy the viscosity and the amine equivalent, provided that when n=0, at least one X is A, and that octamethyl cyclotetrasiloxane (D4), decamethyl cyclopentasiloxane (D5), and dodecamethyl cyclohexasiloxane (D6) are each contained in an amount of less than 3,000 ppm relative to a mass of the component (A);
(B) 5 to 100 mass% of a nonionic surfactant relative to the mass of the component (A);
(C) 50 to 100 mol% of lactic acid or acetic acid relative to a number of moles of amino groups of the component (A); and
(D) 85 to 10,000 mass% of water relative to the mass of the component (A),
   wherein an increased amount of each of (D4), (D5), and (D6) is less than 3,000 ppm relative to the mass of the component (A) when an emulsion of the amino-modified silicone has been stored at 25°C for 6 months.

Hereinafter, the present invention will be described in detail, but the present invention is not limited thereto.

### [Amino-Modified Silicone Emulsion]

The inventive amino-modified silicone emulsion contains:
(A) an amino-modified silicone represented by the general formula (1) described below;
(B) 5 to 100 mass% of a nonionic surfactant relative to the mass of the component (A);
(C) 50 to 100 mol% of lactic acid or acetic acid relative to a number of moles of amino groups of the component (A); and
(D) 85 to 10,000 mass% of water relative to the mass of the component (A),
   where an increased amount of each of octamethyl cyclotetrasiloxane (D4), decamethyl cyclopentasiloxane (D5), and dodecamethyl cyclohexasiloxane (D6) is less than 3,000 ppm relative to the mass of the component (A) when the amino-modified silicone emulsion has been stored at 25°C for 6 months.

### [(A) Amino-Modified Silicone]

The component (A) amino-modified silicone is represented by the following general formula (1), has a viscosity at 25°C of 200 to 100,000 mPa·s, and has an amine equivalent of 1,000 to 20,000 g/mol.

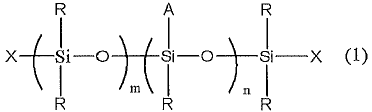

In the formula, R represents a substituted or unsubstituted monovalent hydrocarbon group having 1 to 12 carbon atoms, A represents a monovalent group shown by -R¹(NR²CH₂CH₂)ₐNR³R⁴, where R¹ represents a divalent hydrocarbon group having 1 to 8 carbon atoms, R², R³, and R⁴ each independently represent a hydrogen atom or a monovalent hydrocarbon group having 1 to 4 carbon atoms, and "a" represents an integer of 0 to 4, X represents a monovalent group shown by R, A, or -OR⁵, R⁵ represents a hydrogen atom or a monovalent hydrocarbon group having 1 to 8 carbon atoms, and "m" and "n" are mean values and are numbers that satisfy the viscosity and the amine equivalent, provided that when n=0, at least one X is A, and that octamethyl cyclotetrasiloxane (D4), decamethyl cyclopentasiloxane (D5), and dodecamethyl cyclohexasiloxane (D6) are each contained in an amount of less than 3,000 ppm relative to a mass of the component (A).

In the formula (1), R represents a substituted or unsubstituted monovalent hydrocarbon group having 1 to 12, preferably 1 to 8 carbon atoms, and examples thereof include: alkyl groups such as a methyl group, an ethyl group, a propyl group, a butyl group, an isobutyl group, a pentyl group, a hexyl group, a heptyl group, an octyl group, and a decyl group; alkenyl groups such as a vinyl group, an allyl group, a propenyl group, and a butenyl group; cycloalkyl groups such as a cyclobutyl group, a cyclopentyl group, and a cyclohexyl group; aryl groups such as a phenyl group and a tolyl group; aralkyl groups such as a benzyl group and a phenethyl group; and groups obtained by substituting some or all of the hydrogen atoms bonded to the carbon atoms of these groups with halogen atoms of chlorine, fluorine, bromine, iodine, or the like. In particular, a methyl group is particularly preferable in view of providing hair with smoothness.

In the formula (1), A represents a monovalent group shown by -R¹(NR²CH₂CH₂)ₐNR³R⁴, and R¹ represents a divalent hydrocarbon group having 1 to 8, preferably 2 to 4 carbon atoms. As R¹, an alkylene group such as a methylene group, a dimethylene group, a trimethylene group, or a tetramethylene group is preferable, and in particular, a trimethylene group is preferable. In addition, "a" represents an integer of 0 to 4. R², R³, and R⁴ each independently represent a hydrogen atom or a monovalent hydrocarbon group having 1 to 4 carbon atoms. Examples of the hydrocarbon group include a methyl group, an ethyl group, a propyl group, a butyl group, and an isobutyl group. A hydrogen atom is preferable.

Examples of A include the following.

- (CH₂)₃NH₂

- (CH₂)₃NHCH₂CH₂NH₂

- (CH₂)₃NHCH₂CH₂NHCH₂CH₂NH₂

-CH₂CH(CH₃)CH₂NHCH₂CH₂NH₂

In particular, a 3-aminopropyl group represented by -(CH₂)₃NH₂ and an N-(2-aminoethyl)-3-aminopropyl group represented by -(CH₂)₃NHCH₂CH₂NH₂ are preferable since hair and fiber can be provided with smoothness and softness.

The component (A) has a viscosity at 25°C of 200 to 100,000 mPa·s, preferably 500 to 10,000 mPa·s, more preferably 800 to 3,000 mPa·s. If the viscosity at 25°C is less than the lower limit, adsorption to hair is poor, and conditioning effects are insufficient. In addition, emulsification is difficult. If the viscosity at 25°C exceeds the upper limit, there is risk that the particle size of the inventive amino-modified silicone emulsion cannot be easily made fine, and that concentration separation occurs when blended in a hair cosmetic or a softener (fiber treatment agent).

Furthermore, the component (A) has an amine equivalent of 1,000 to 20,000 g/mol, preferably 1,500 to 10,000 g/mol, more preferably 1,500 to 4,000 g/mol. If the amine equivalent is less than the lower limit, hair may be imparted with stickiness. If the amine equivalent exceeds the upper limit, adsorption to hair tends to be degraded, and durability of conditioning effects tends to be degraded. Note that the amine equivalent is the average molecular weight per nitrogen atom.

The X in the general formula (1) represents a monovalent group shown by the above-described R, A, or - OR⁵. R⁵ represents a hydrogen atom or a monovalent hydrocarbon group having 1 to 8 carbon atoms. Examples of the hydrocarbon group include alkyl groups such as a methyl group, an ethyl group, a propyl group, a butyl group, a pentyl group, a hexyl group, and an octyl group and a phenyl group.

"m" and "n" are mean values and are numbers that satisfy the above-described viscosity and amine equivalent, provided that when "n" is 0, at least one X is A. Typically, 100 ≤ m ≤ 1250 and 1 ≤ n ≤ 50. Note that the arrangement within the parentheses of "m" and "n" in the molecule represented by the formula (1) can be any arrangement.

The cyclic low-molecular-weight siloxanes octamethyl cyclotetrasiloxane (D4), decamethyl cyclopentasiloxane (D5), and dodecamethyl cyclohexasiloxane (D6) are each contained in an amount of less than 3,000 ppm, preferably less than 2,000 ppm, more preferably less than 1,000 ppm relative to the mass of the component (A). If the contained amount of octamethyl cyclotetrasiloxane (D4), decamethyl cyclopentasiloxane (D5), and dodecamethyl cyclohexasiloxane (D6) is the above-described upper limit or more, the amount to be blended may be restricted by legal regulations in some cases where the emulsion is blended in a product which is drained as waste water after use, for example, a hair cosmetic or a fiber treatment agent.

The amino-modified silicone represented by the general formula (1) can be obtained easily by known synthesizing methods. For example, the amino-modified silicone can be obtained by performing an equilibration reaction between a cyclic low-molecular-weight siloxane such as octamethyl cyclotetrasiloxane and a compound selected from 3-aminopropyldiethoxysilane or N-(2-aminoethyl)-3-aminopropylmethyldimethoxysilane, or a hydrolysis product thereof, and hexamethyldisiloxane, etc. as other raw materials in the presence of a catalyst such as an alkali metal hydroxide and tetramethylammonium hydroxide. Note that the component (A) may have a branched unit in the amino group-containing organopolysiloxane skeleton. It is also possible to use a reaction product of the amino-modified silicone represented by the general formula (1) and an organic acid anhydride, a carbonate, an epoxy compound, etc.

In an amino-modified silicone synthesized by an equilibration reaction or the like, octamethyl cyclotetrasiloxane (D4), decamethyl cyclopentasiloxane (D5), and dodecamethyl cyclohexasiloxane (D6) are usually contained in an amount of 3,000 ppm or more each. In order to make octamethyl cyclotetrasiloxane (D4), decamethyl cyclopentasiloxane (D5), and dodecamethyl cyclohexasiloxane (D6) contained in the component (A) less than 3,000 ppm each, the synthesized amino-modified silicone is placed under high temperature and reduced pressure to distil off the (D4), (D5), and (D6) until they are less than 3,000 ppm. Note that when the amount of amine equivalents in the amino-modified silicone is small or when viscosity is low, amino groups are oxidized and the appearance of the emulsion turns yellow in some cases if the temperature of the distilling-off under reduced pressure is 130°C or higher. In such a case, yellowing of the appearance can be suppressed by distilling off under reduced pressure at 100 to 130°C.

Besides the amino-modified silicone, other silicone oils can also be contained. Examples thereof include dimethyl silicone, epoxy-modified silicone, hydroxy-modified silicone, polyether-modified silicone, acryl-modified silicone, methacryl-modified silicone, mercapto-modified silicone, carbinol-modified silicone, methylphenyl silicone, methylhydrogen silicone, phenol-modified silicone, and alkyl-modified silicone. In particular, smoothness of hair can be improved by combining with dimethyl silicone having a high polymerization degree. In addition, by combining with epoxy-modified silicone or hydroxy-modified silicone, a film is formed, so that smoothness of the hair or fiber lasts longer.

### [(B) Nonionic Surfactant]

Examples of the nonionic surfactant include a polyoxyalkylene alkyl ether such as polyoxyethylene alkyl ether, polyoxyethylene polyoxypropylene alkyl ether, polyoxyethylene alkyl phenyl ether, polyoxyethylene fatty acid ester, sorbitan fatty acid ester, polyoxyethylene sorbitan fatty acid ester, and glycerin fatty acid ester. Specific examples thereof include polyoxyethylene octyl ether, polyoxyethylene polyoxypropylene octyl ether, polyoxyethylene nonyl ether, polyoxyethylene decyl ether, polyoxyethylene polyoxypropylene decyl ether, polyoxyethylene lauryl ether, polyoxyethylene polyoxypropylene lauryl ether, polyoxyethylene tridecyl ether, polyoxyethylene polyoxypropylene tridecyl ether, and polyoxyethylene cetyl ether. It is also possible to use a reactive surfactant having a functional group.

The component (B) may be a mixture of two or more nonionic surfactants having different HLB values. When nonionic surfactants are combined so that the difference between the HLB values of at least two polyoxyalkylene alkyl ethers is 1 or more, preferably 2 or more, and the HLB value of the whole mixture is within the range of 12 to 16, the stability of the inventive amino-modified silicone emulsion is raised, and concentration separation tends to occur less.

The blended amount of the component (B) is 5 to 100 mass%, preferably 10 to 50 mass% relative to the mass of the component (A). If the amount is less than the lower limit, the particle size of the obtained emulsion is large, and there is risk of concentration separation over time. Meanwhile, if the amount is greater than the upper limit, the viscosity of the inventive amino-modified silicone emulsion at 25°C is high, so that handling is harder.

### [(C) Lactic Acid or Acetic Acid]

The component (C) is lactic acid or acetic acid. Alternatively, lactic acid and acetic acid may be mixed together at any ratio and blended in the emulsion. The component (C) may be blended over multiple times as in the Examples described below.

When a specific amount of an organic acid is blended in the amino-modified silicone emulsion, the pH of the emulsion stabilizes. In addition, when an organic acid is blended, the particle size of the emulsion becomes small more easily, and an organic acid is blended in many amino-modified silicone emulsions.

The increased amount of octamethyl cyclotetrasiloxane (D4), decamethyl cyclopentasiloxane (D5), and dodecamethyl cyclohexasiloxane (D6) in the amino-modified silicone emulsion over time varies depending on the type and blended amount of the blended organic acid. As a result of earnest study on the present invention, it has been found that increase in octamethyl cyclotetrasiloxane (D4), decamethyl cyclopentasiloxane (D5), and dodecamethyl cyclohexasiloxane (D6) in the emulsion over time can be suppressed by blending a specific amount of acetic acid or lactic acid in the amino-modified silicone emulsion. Although dependent on the structure of the amino-modified silicone, out of acetic acid and lactic acid, lactic acid has a higher effect of suppressing the increase in octamethyl cyclotetrasiloxane (D4), decamethyl cyclopentasiloxane (D5), and dodecamethyl cyclohexasiloxane (D6) in some cases.

The amino-modified silicone emulsion turns yellow depending on the type and blended amount of the blended organic acid. Since yellowing occurs the least particularly when lactic acid, among organic acids, is blended, lactic acid is more preferably blended.

The component (C) is blended in an amount of 50 to 100 mol%, preferably 60 to 90 mol%, more preferably 65 to 90 mol% relative to the number of moles of amino groups of the component (A). If the amount is less than the lower limit, there is risk of the pH of the inventive amino-modified silicone emulsion rising over time. In addition, there is risk of the increased amount of octamethyl cyclotetrasiloxane (D4), decamethyl cyclopentasiloxane (D5), and dodecamethyl cyclohexasiloxane (D6) in the amino-modified silicone emulsion over time increasing. On the other hand, if the amount exceeds the upper limit, there is risk of the amino-modified silicone emulsion turning yellow or the increased amount of octamethyl cyclotetrasiloxane (D4), decamethyl cyclopentasiloxane (D5), and dodecamethyl cyclohexasiloxane (D6) in the amino-modified silicone emulsion over time increasing.

### [(D) Water]

The component (D) is blended in an amount of 85 to 10,000 mass%, preferably 85 to 500 mass% relative to the mass of the component (A). If the amount is less than the lower limit, there is risk of the inventive amino-modified silicone emulsion becoming viscous, and becoming difficult to handle. On the other hand, if the amount exceeds the upper limit, the proportion of the amino-modified silicone in the emulsion is small, so that there is risk that effects such as smoothness and softness cannot be exhibited when the inventive amino-modified silicone emulsion is blended in a hair cosmetic or a softener.

The pH of the inventive amino-modified silicone emulsion at 25°C can be 4.5 to 7.0, preferably 5.5 to 7.0, and a pH of 5.5 to 6.5 is more preferable. Here, the pH is measured immediately after the emulsion has been manufactured. The increase in octamethyl cyclotetrasiloxane (D4), decamethyl cyclopentasiloxane (D5), and dodecamethyl cyclohexasiloxane (D6) in the amino-modified silicone emulsion over time can be suppressed when the pH is within the above ranges, and the increase can be suppressed further when the pH is within the range of 5.5 to 6.5. When the pH is the lower limit or higher, the risk of the inventive amino-modified silicone emulsion yellowing is reduced. On the other hand, when the pH is the upper limit or lower, the risk of the pH of the inventive amino-modified silicone emulsion rising over time is reduced.

The increased amount of each of octamethyl cyclotetrasiloxane (D4), decamethyl cyclopentasiloxane (D5), and dodecamethyl cyclohexasiloxane (D6) is less than 3,000 ppm, preferably less than 2,000 ppm, more preferably 1,000 ppm relative to the mass of the organopolysiloxane (A) when the inventive amino-modified silicone emulsion has been stored at 25°C for 6 months. If the increased amount of each of octamethyl cyclotetrasiloxane (D4), decamethyl cyclopentasiloxane (D5), and dodecamethyl cyclohexasiloxane (D6) is the upper limit or more, there is risk that the amount to be blended may be restricted by legal regulations when the inventive amino-modified silicone emulsion is blended in a product for use in a hair cosmetic, a softener, etc., which are drained as waste water after use.

### [(E) Polyhydric Alcohol]

The component (E) is an optional component of the inventive amino-modified silicone emulsion, and when the component (E) is blended, the inventive amino-modified silicone emulsion becomes less viscous and easier to handle in some cases, and the increase in octamethyl cyclotetrasiloxane (D4), decamethyl cyclopentasiloxane (D5), and dodecamethyl cyclohexasiloxane (D6) over time in the amino-modified silicone emulsion can be suppressed in some cases. In addition, when the inventive amino-modified silicone emulsion containing the component (E) is blended in a hair cosmetic or a softener, moisturizing property can be provided. The component (E) is preferably contained in an amount of 1 to 20 mass% relative to the mass of the component (A).

The component (E) can be appropriately selected from known polyhydric alcohols. Examples thereof include diols such as dipropylene glycol, ethylene glycol, glycerin, diglycerin, isopropylene glycol, propylene glycol, 1,3-butylene glycol, 1,2-propylene glycol, 1,3-propylene glycol, dipropylene glycol, 1,2-butylene glycol, 1,3-butylene glycol, 1,4-butylene glycol, 2,3-butylene glycol, 1,5-pentanediol, 2-methyl-2,4-pentanediol, 1,6-hexanediol, polyethylene glycol, and polypropylene glycol; alicyclic diols such as 1,2-cyclohexanediol, 1,3-cyclohexanediol, 1,4-cyclohexanediol, and 1,2-di(hydroxymethyl)cyclohexanone; aromatic diols such as 1,2-di(hydroxymethyl)benzene; alkanepolyols such as pentaerithritol and glycerin; saccharide derivatives such as sorbitol and mannitol; polyglycerins such as pentaglycerin and hexaglycerin; polyalkanepolyols such as polypentaerithritol; and the like. One of these may be used or a mixture of two or more thereof may be used. In particular, ethylene glycol, glycerin, 1,2-propanediol, 1,3-propanediol, 1,2-butylene glycol, 1,3-butylene glycol, 1,4-butylene glycol, 2,3-butylene glycol, 1,5-pentanediol, and 2-methyl pentane-2,4-diol are preferable.

### [(F) Anionic Surfactant]

The component (F) is an optional component of the inventive amino-modified silicone emulsion. By blending the component (F), the inventive amino-modified silicone emulsion becomes less viscous and easier to handle in some cases. The component (F) is preferably contained in an amount of 0.1 to 3 mass% relative to the mass of the component (A).

The component (F) can be appropriately selected from known anionic surfactants. Examples thereof include alkyl sulfates, alkyl sulfate ester salts, alkylbenzene sulfonates and salts thereof, sulfate ester salts of monoalkyl polyoxyethylene ethers, acetic acid ester salts of monoalkyl polyoxyethylene ethers, alkylnaphthylsulfonates and salts thereof, alkali metal sulforicinates, alkali metal sulfosuccinates, alkyl phosphates and salts thereof, phosphate ester salts of monoalkyl polyoxyethylene ethers, and sulfonated glyceryl esters of fatty acids. One of these may be used or a mixture of two or more thereof may be used. Specific examples thereof include lauryl sulfate, sodium lauryl sulfate, triethanol amine lauryl sulfate, ammonium lauryl sulfate, sodium polyoxyethylene lauryl ether sulfate, sodium polyoxyethylene lauryl ether acetate, sodium dodecylbenzenesulfonate, lauryl 2 sodium polyoxyethylene sulfosuccinate, sodium dioctylsulfosuccinate, sodium polyoxyethylene lauryl ether phosphoric acid ester, and sodium alkyl naphthalene sulfonate. It is also possible to use a reactive surfactant having a functional group.

The viscosity of the inventive amino-modified silicone emulsion at 25°C can be 30,000 mPa·s or lower, preferably 10,000 mPa·s or lower, more preferably 5,000 mPa·s or lower. Here, the viscosity is measured immediately after the emulsion has been manufactured. When the viscosity at 25°C is the upper limit or lower, handling and blending in a hair cosmetic or a softener are easier. The viscosity can be measured using a BM-type viscometer (manufactured by Toki Sangyo Co., Ltd.).

The average particle size of the inventive amino-modified silicone emulsion can be 1,000 nm or less, preferably 600 nm or less, more preferably 400 nm or less. When the average particle size is less than 1,000 nm, there is little risk of stability being degraded and oil floating or the like occurring during dilution or stirring. In addition, when stored for a long period, concentration separation hardly occurs. The average particle size of the emulsion can be measured with LA920 or LA960 manufactured by Horiba Ltd. or N4 PLUS or Delsa Max manufactured by BECKMAN COULTER.

### [Preparation of Emulsion Composition]

Methods for manufacturing the inventive amino-modified silicone emulsion may follow conventionally known methods. Specific examples of preparation methods are mainly as shown below.

The component (A) amino-modified silicone, the component (B) nonionic surfactant, the component (C) lactic acid or acetic acid, a part of the component (D) water, and as necessary, the component (E) polyhydric alcohol and the component (F) anionic surfactant are mixed, emulsified, and stirred under shearing force. Subsequently, this is diluted with the rest of the component (D) water to give the target amino-modified silicone emulsion.

Alternatively, when the emulsification of the component (A) is difficult, the component (A), the component (B), and a part of the component (D) can be mixed, emulsified, and stirred under shearing force. Subsequently, this can be diluted with the rest of the component (D) water to obtain an emulsion, and then the component (C) and as necessary, the component (E) and the component (F) can be blended in the diluted emulsion and stirred to achieve the target amino-modified silicone emulsion. An example of details of an emulsification method is as follows.

The component (A) amino-modified silicone, the component (B) nonionic surfactant, the component (C) lactic acid or acetic acid, a part of the component (D) water, and as necessary, the component (E) polyhydric alcohol and the component (F) anionic surfactant are blended in Combi Mix (PRIMIX Corporation), and the mixture is emulsified with a homomixer (a stirrer employing the rotation of a rotor inside a stator) at 2,000 rpm or a disperser (a stirrer employing the rotation of tooth-form blades) at 2,000 rpm, and an anchor at 20 rpm. After the entire mixture is emulsified, the resultant is stirred for 15 to 180 minutes with a disperser at 2,000 rpm and an anchor at 30 rpm until a predetermined particle size is achieved. Subsequently, the remaining component (D) water is added to dilute the resultant with the homomixer at 2,000 to 3,000 rpm. Thus, the inventive amino-modified silicone emulsion is prepared.

The temperature on emulsification is not particularly specified, but is preferably 0 to 80°C, more preferably 10 to 60°C. Emulsification is easily carried out at a temperature of 10°C to 60°C, and the produced emulsion tends to be more stable. When an amino-modified silicone emulsion is heated at 70 to 80°C for 1 to 30 hours, the particle size becomes smaller or viscosity is lowered in some cases. Therefore, a step of heating at 70 to 80°C may be added in the production of an amino-modified silicone emulsion according to the target particle size or viscosity. During emulsification, the pressure can be not only normal pressure, but also reduced pressure or raised pressure. When emulsification is performed under reduced pressure or under pressure, it becomes difficult for bubbles to get mixed in, and emulsification can be performed effectively. When the pressure is to be reduced, the pressure is preferably higher than the vapor pressure of the raw materials, so that the raw materials do not volatilize. In addition, the emulsification time is not particularly specified, and can be the time at which the target particle size is achieved, but is generally preferably 30 to 360 minutes.

The emulsifying machine for emulsification is not particularly limited as long as the raw materials and emulsified composition can be stirred. It is possible to use a colloid mill having a stirring unit including a rotor and a stator (IKA, PUC, NIHONSEIKI KAISHA LTD., and IWAKI & CO., LTD.), a high shear mixer (Silverson and PRIMIX Corporation), a HOMOGENIZING DISPER (PRIMIX Corporation), AGI HOMO MIXER (PRIMIX Corporation), Combi Mix (PRIMIX Corporation) being a 3-axis disperser-kneader having a combination of a homomixer, a homogenizing disper, and an anchor mixer, HAAKE Mini LabII (Thermo scientific) being a 2-axis mixer having same-direction screws or different-direction screws, MC15 and MC5 (Rheo Lab), etc.

In the present invention, the cyclic low-molecular-weight siloxanes (octamethyl cyclotetrasiloxane (D4), decamethyl cyclopentasiloxane (D5), and dodecamethyl cyclohexasiloxane (D6)) in an amino-modified silicone and an amino-modified silicone emulsion can be analyzed by employing gas chromatography. Specifically, a small amount of the amino-modified silicone or the amino-modified silicone emulsion is added to an organic solvent (for example, acetone or hexane) that can dissolve the cyclic low-molecular-weight siloxanes, and the mixture is shaken for about 30 minutes to 2 hours. After extracting the cyclic low-molecular-weight siloxanes into the organic solvent by shaking, the organic solvent containing the cyclic low-molecular-weight siloxanes is assessed by gas chromatography (Agilent 7890B (manufactured by Agilent Technologies Japan, Ltd.)).

Besides the cationic surfactant, it is possible to blend in the inventive amino-modified silicone emulsion, a water-soluble polymer such as a polyvinyl alcohol, methyl cellulose, carboxymethyl cellulose, hydroxymethyl cellulose, polyvinyl pyrrolidone, an alginate salt, xanthan gum, and an acrylic acid polymer as a protective colloid or a thickener in accordance with the purpose of the present invention. Furthermore, it is also possible to blend an antibacterial agent or antiseptic such as an oxazoline-based compound or an aromatic carboxylic acid salt, a fragrance, an antioxidant, an anticorrosive agent, a dye, a filler, a curing catalyst, an organic powder, an inorganic powder, etc.

### [Hair Cosmetic]

The amino-modified silicone emulsion obtained in the above-described manner is extremely useful for hair cosmetics such as shampoos and conditioners. The blended amount of the amino-modified silicone emulsion is preferably 0.1 to 20 mass% of the hair cosmetic, for example, and is further preferably 0.5 to 10 mass%. When the blended amount is the lower limit or more, conditioning effects on hair can be achieved sufficiently, and when the amount is the upper limit or less, there is no stickiness, and favorable feeling can be achieved.

### [Fiber Treatment Agent]

The inventive amino-modified silicone emulsion is also extremely useful for fiber treatment agents such as softeners and washing detergents. For example, the amino-modified silicone emulsion can be blended in an amount of 0.1 to 20 mass% of the softener, preferably 0.5 to 10 mass%. When the blended amount is the lower limit or more, there is little risk of texture being degraded. On the other hand, when the blended amount is the upper limit or less, there is little risk of yellowing during storage.

According to the present invention, in an amino-modified silicone emulsion obtained by emulsifying an amino-modified silicone with a nonionic surfactant and a specific organic acid (lactic acid or acetic acid), and if needed, a polyhydric alcohol and an anionic surfactant, octamethyl cyclotetrasiloxane (D4), decamethyl cyclopentasiloxane (D5), and dodecamethyl cyclohexasiloxane (D6) hardly increase during storage. Moreover, when the amino-modified silicone emulsion is blended in a hair cosmetic or a fiber treatment agent, excellent smoothness and softness are provided.

### EXAMPLE

Hereinafter, the present invention will be described specifically with reference to Examples and Comparative Examples. However, the present invention is not restricted to the following Examples.

### [Example 1]

30 parts by mass (90 g) of a component (A) [1] amino-modified silicone (viscosity: 800 mPa·s, amine equivalent: 1,500 g/mol) (1a) shown by the following formula (2), 5.0 parts by mass (15 g) of a component (B) [1] polyoxyethylene alkyl (having 12 to 14 carbon atoms) ether (12 EO) (SANNONIC SS-120, manufactured by Sanyo Chemical Industries, Ltd.) (2a), 2.0 parts by mass (6 g) of a component (E) [1] 1,3-butylene glycol (manufactured by KH Neochem Co., Ltd.) (4a), and 13.5 parts by mass (40.5 g) of a component (D) [1]-1 water were charged into HOMOGENIZING MIXER MARK II Model 2.5 (manufactured by PRIMIX Corporation) and stirred at 500 rpm. When the entire mixture emulsified, the resultant was stirred with HOMOGENIZING DISPER Model 2.5 (manufactured by PRIMIX Corporation) at 1,500 rpm for 25 minutes. Subsequently, 46.7 parts by mass (140.1 g) of the component (D) [1]-2 water was added, and the resultant was stirred with HOMOGENIZING MIXER MARK II Model 2.5 (manufactured by PRIMIX Corporation) at 1,500 rpm for 3 minutes for dilution. After that, 1.8 parts by mass (5.4 g) of a component (C) [1]-2 lactic acid (90% aqueous solution, manufactured by Musashino Chemical Laboratory, Ltd.) (3a) was blended, and the resultant was stirred with HOMOGENIZING MIXER MARK II Model 2.5 (manufactured by PRIMIX Corporation) at 1,500 rpm for 3 minutes. Next, 1.0 parts by mass (3 g) of phenoxy ethanol was blended as an antiseptic, and the resulting mixture was stirred with HOMOGENIZING MIXER MARK II Model 2.5 (manufactured by PRIMIX Corporation) at 1,500 rpm for 3 minutes. Thus, an emulsion 1 was prepared. 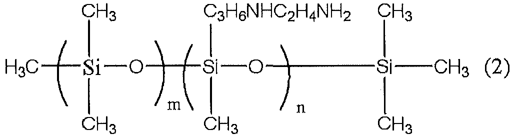 (m = 200, n = 5)

### [Example 2]

30 parts by mass (90 g) of the component (A) [1] amino-modified silicone (viscosity: 800 mPa·s, amine equivalent: 1,500 g/mol) (1a) shown by the formula (2), 5.0 parts by mass (15 g) of the component (B) [1] polyoxyethylene alkyl (having 12 to 14 carbon atoms) ether (12 EO) (SANNONIC SS-120, manufactured by Sanyo Chemical Industries, Ltd.) (2a), 2.0 parts by mass (6.0 g) of the component (E) [1] 1,3-butylene glycol (manufactured by KH Neochem Co., Ltd.) (4a), and 13.5 parts by mass (40.5 g) of the component (D) [1]-1 water were charged into HOMOGENIZING MIXER MARK II Model 2.5 (manufactured by PRIMIX Corporation) and stirred at 500 rpm. When the entire mixture emulsified, the resultant was stirred with HOMOGENIZING DISPER Model 2.5 (manufactured by PRIMIX Corporation) at 1,500 rpm for 25 minutes. Subsequently, 47.2 parts by mass (141.6 g) of the component (D) [1]-2 water was added, and the resultant was stirred with HOMOGENIZING MIXER MARK II Model 2.5 (manufactured by PRIMIX Corporation) at 1,500 rpm for 3 minutes for dilution. After that, 1.3 parts by mass (3.9 g) of the component (C) [1]-2 lactic acid (90% aqueous solution, manufactured by Musashino Chemical Laboratory, Ltd.) (3a) was blended, and the resultant was stirred with HOMOGENIZING MIXER MARK II Model 2.5 (manufactured by PRIMIX Corporation) at 1,500 rpm for 3 minutes. Next, 1.0 parts by mass (3 g) of phenoxy ethanol was blended as an antiseptic, and the resulting mixture was stirred with HOMOGENIZING MIXER MARK II Model 2.5 (manufactured by PRIMIX Corporation) at 1,500 rpm for 3 minutes. Thus, an emulsion 2 was prepared.

### [Example 3]

30 parts by mass (90 g) of the component (A) [1] amino-modified silicone (viscosity: 800 mPa·s, amine equivalent: 1,500 g/mol) (1a) shown by the formula (2), 5.0 parts by mass (15 g) of the component (B) [1] polyoxyethylene alkyl (having 12 to 14 carbon atoms) ether (12 EO) (SANNONIC SS-120, manufactured by Sanyo Chemical Industries, Ltd.) (2a), 2.0 parts by mass (6.0 g) of the component (E) [1] 1,3-butylene glycol (manufactured by KH Neochem Co., Ltd.) (4a), and 13.5 parts by mass (40.5 g) of the component (D) [1]-1 water were charged into HOMOGENIZING MIXER MARK II Model 2.5 (manufactured by PRIMIX Corporation) and stirred at 500 rpm. When the entire mixture emulsified, the resultant was stirred with HOMOGENIZING DISPER Model 2.5 (manufactured by PRIMIX Corporation) at 1,500 rpm for 25 minutes. Subsequently, 45.9 parts by mass (137.7 g) of the component (D) [1]-2 water was added, and the resultant was stirred with HOMOGENIZING MIXER MARK II Model 2.5 (manufactured by PRIMIX Corporation) at 1,500 rpm for 3 minutes for dilution. After that, 2.6 parts by mass (7.8 g) of a component (C) [2]-2 acetic acid (30% aqueous solution, manufactured by Kozakai Pharmaceutical Co., Ltd.) (3b) was blended, and the resultant was stirred with HOMOGENIZING MIXER MARK II Model 2.5 (manufactured by PRIMIX Corporation) at 1,500 rpm for 3 minutes. Next, 1.0 parts by mass (3 g) of phenoxy ethanol was blended as an antiseptic, and the resulting mixture was stirred with HOMOGENIZING MIXER MARK II Model 2.5 (manufactured by PRIMIX Corporation) at 1,500 rpm for 3 minutes. Thus, an emulsion 3 was prepared.

### [Example 4]

50 parts by mass (150 g) of a component (A) [2] amino-modified silicone (viscosity: 80,000 mPa·s, amine equivalent: 20,000 g/mol) (1a) shown by the following formula (3), 1.0 parts by mass (3 g) of a component (B) [2] polyoxyethylene alkyl (having 12 carbon atoms) ether (4 EO) (LEOX CL-40, manufactured by Lion Corporation) (2b), 4.0 parts by mass (12 g) of a component (B) [3] polyoxyethylene alkyl (having 12 carbon atoms) ether (23 EO) (LEOX CL-230, manufactured by Lion Corporation) (2c), 0.5 parts by mass (1.5 g) of a component (F) [1] sodium polyoxyethylene lauryl ether sulfate (3 EO) (25% aqueous solution, EMAL 20C, manufactured by Kao Corporation) (5a), and 5.5 parts by mass (16.5 g) of the component (D) [1]-1 water were charged into HOMOGENIZING DISPER Model 2.5 (manufactured by PRIMIX Corporation) and stirred at 1,500 rpm for 40 minutes. Subsequently, 37.55 parts by mass (112.65 g) of the component (D) [1]-2 water was added, and the resultant was stirred with HOMOGENIZING MIXER MARK II Model 2.5 (manufactured by PRIMIX Corporation) at 1,500 rpm for 3 minutes for dilution. After that, 0.45 parts by mass (1.35 g) of the component (C) [2]-2 acetic acid (30% aqueous solution, manufactured by Kozakai Pharmaceutical Co., Ltd.) (3b) was blended, and the resultant was stirred with HOMOGENIZING MIXER MARK II Model 2.5 (manufactured by PRIMIX Corporation) at 1,500 rpm for 3 minutes. Next, 1.0 parts by mass (3 g) of phenoxy ethanol was blended as an antiseptic, and the resulting mixture was stirred with HOMOGENIZING MIXER MARK II Model 2.5 (manufactured by PRIMIX Corporation) at 1,500 rpm for 3 minutes. Thus, an emulsion 4 was prepared. 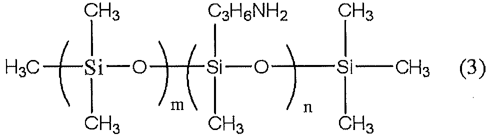 (m = 1300, n = 5)

### [Example 5]

30 parts by mass (90 g) of the component (A) [1] amino-modified silicone (viscosity: 800 mPa·s, amine equivalent: 1,500 g/mol) (1a) shown by the formula (2), 5.0 parts by mass (15 g) of the component (B) [1] polyoxyethylene alkyl (having 12 to 14 carbon atoms) ether (12 EO) (SANNONIC SS-120, manufactured by Sanyo Chemical Industries, Ltd.) (2a), 0.38 parts by mass (1.14 g) of a component (C) [1]-1 lactic acid (90% aqueous solution, manufactured by Musashino Chemical Laboratory, Ltd.) (3a), 4.0 parts by mass (12 g) of the component (E) [1] 1,3-butylene glycol (manufactured by KH Neochem Co., Ltd.) (4a), and 12 parts by mass (36 g) of the component (D) [1]-1 water were charged into HOMOGENIZING MIXER MARK II Model 2.5 (manufactured by PRIMIX Corporation) and stirred at 500 rpm. When the entire mixture emulsified, the resultant was stirred with HOMOGENIZING MIXER MARK II Model 2.5 (manufactured by PRIMIX Corporation) at 5,000 rpm for 25 minutes. Subsequently, 46.7 parts by mass (140.1 g) of the component (D) [1]-2 water was added, and the resultant was stirred with HOMOGENIZING MIXER MARK II Model 2.5 (manufactured by PRIMIX Corporation) at 1,500 rpm for 3 minutes for dilution. After that, 0.92 parts by mass (2.76 g) of the component (C) [1]-2 lactic acid (90% aqueous solution, manufactured by Musashino Chemical Laboratory, Ltd.) (3a) was blended, and the resultant was stirred with HOMOGENIZING MIXER MARK II Model 2.5 (manufactured by PRIMIX Corporation) at 1,500 rpm for 3 minutes. Next, 1.0 parts by mass (3 g) of phenoxy ethanol was blended as an antiseptic, and the resulting mixture was stirred with HOMOGENIZING MIXER MARK II Model 2.5 (manufactured by PRIMIX Corporation) at 1,500 rpm for 3 minutes. Thus, an emulsion 5 was prepared.

### [Example 6]

30 parts by mass (90 g) of a component (A) [3] amino-modified silicone (viscosity: 1,800 mPa·s, amine equivalent: 3,500 g/mol) (1c) shown by the following formula (4), 10 parts by mass (30 g) of the component (B) [1] polyoxyethylene alkyl (having 12 to 14 carbon atoms) ether (12 EO) (SANNONIC SS-120, manufactured by Sanyo Chemical Industries, Ltd.) (2a), 0.6 parts by mass (1.8 g) of the component (C) [1]-1 lactic acid (90% aqueous solution, manufactured by Musashino Chemical Laboratory, Ltd.) (3a), 4.0 parts by mass (12 g) of the component (E) [1] 1,3-butylene glycol (manufactured by KH Neochem Co., Ltd.) (4a), 0.5 parts by mass (1.5 g) of the component (F) [1] sodium polyoxyethylene lauryl ether sulfate (3 EO) (25% aqueous solution, EMAL 20C, manufactured by Kao Corporation) (5a), and 12 parts by mass (36 g) of the component (D) [1]-1 water were charged into HOMOGENIZING MIXER MARK II Model 2.5 (manufactured by PRIMIX Corporation) in two portions and stirred at 500 rpm. When the entire mixture emulsified, the resultant was stirred with HOMOGENIZING MIXER MARK II Model 2.5 (manufactured by PRIMIX Corporation) at 5,000 rpm for 25 minutes. Subsequently, 41.9 parts by mass (125.7 g) of the component (D) [1]-2 water was added, and the resultant was stirred with HOMOGENIZING MIXER MARK II Model 2.5 (manufactured by PRIMIX Corporation) at 1,500 rpm for 3 minutes for dilution. Next, 1.0 parts by mass (3 g) of phenoxy ethanol was blended as an antiseptic, and the resulting mixture was stirred with HOMOGENIZING MIXER MARK II Model 2.5 (manufactured by PRIMIX Corporation) at 1,500 rpm for 3 minutes. Thus, an emulsion 6 was prepared. 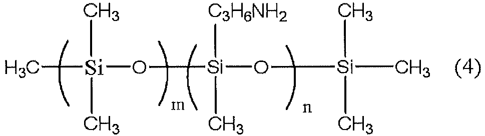 (m = 450, n = 10)

### [Comparative Example 1]

30 parts by mass (90 g) of the component (A) [1] amino-modified silicone (viscosity: 800 mPa·s, amine equivalent: 1,500 g/mol) (1a) shown by the formula (2), 5.0 parts by mass (15 g) of the component (B) [1] polyoxyethylene alkyl (having 12 to 14 carbon atoms) ether (12 EO) (SANNONIC SS-120, manufactured by Sanyo Chemical Industries, Ltd.) (2a), 2.0 parts by mass (6.0 g) of the component (E) [1] 1,3-butylene glycol (manufactured by KH Neochem Co., Ltd.) (4a), and 13.5 parts by mass (40.5 g) of the component (D) [1]-1 water were charged into HOMOGENIZING MIXER MARK II Model 2.5 (manufactured by PRIMIX Corporation) and stirred at 500 rpm. When the entire mixture emulsified, the resultant was stirred with HOMOGENIZING DISPER Model 2.5 (manufactured by PRIMIX Corporation) at 1,500 rpm for 25 minutes. Subsequently, 47.9 parts by mass (143.7 g) of the component (D) [1]-2 water was added, and the resultant was stirred with HOMOGENIZING MIXER MARK II Model 2.5 (manufactured by PRIMIX Corporation) at 1,500 rpm for 3 minutes for dilution. After that, 0.6 parts by mass (1.8 g) of the component (C) [1]-2 lactic acid (90% aqueous solution, manufactured by Musashino Chemical Laboratory, Ltd.) (3a) was blended, and the resultant was stirred with HOMOGENIZING MIXER MARK II Model 2.5 (manufactured by PRIMIX Corporation) at 1,500 rpm for 3 minutes. Next, 1.0 parts by mass (3 g) of phenoxy ethanol was blended as an antiseptic, and the resulting mixture was stirred with HOMOGENIZING MIXER MARK II Model 2.5 (manufactured by PRIMIX Corporation) at 1,500 rpm for 3 minutes. Thus, an emulsion 7 was prepared.

### [Comparative Example 2]

30 parts by mass (90 g) of the component (A) [1] amino-modified silicone (viscosity: 800 mPa·s, amine equivalent: 1,500 g/mol) (1a) shown by the formula (2), 5.0 parts by mass (15 g) of the component (B) [1] polyoxyethylene alkyl (having 12 to 14 carbon atoms) ether (12 EO) (SANNONIC SS-120, manufactured by Sanyo Chemical Industries, Ltd.) (2a), 2.0 parts by mass (6.0 g) of the component (E) [1] 1,3-butylene glycol (manufactured by KH Neochem Co., Ltd.) (4a), and 13.5 parts by mass (40.5 g) of the component (D) [1]-1 water were charged into HOMOGENIZING MIXER MARK II Model 2.5 (manufactured by PRIMIX Corporation) and stirred at 500 rpm. When the entire mixture emulsified, the resultant was stirred with HOMOGENIZING DISPER Model 2.5 (manufactured by PRIMIX Corporation) at 1,500 rpm for 25 minutes. Subsequently, 47.54 parts by mass (142.62 g) of the component (D) [1]-2 water was added, and the resultant was stirred with HOMOGENIZING MIXER MARK II Model 2.5 (manufactured by PRIMIX Corporation) at 1,500 rpm for 3 minutes for dilution. After that, 0.96 parts by mass (2.88 g) of an acid component [3]-2 propionic acid (manufactured by Kanto Chemical Co., Inc.) (3c) was blended, and the resultant was stirred with HOMOGENIZING MIXER MARK II Model 2.5 (manufactured by PRIMIX Corporation) at 1,500 rpm for 3 minutes. Next, 1.0 parts by mass (3 g) of phenoxy ethanol was blended as an antiseptic, and the resulting mixture was stirred with HOMOGENIZING MIXER MARK II Model 2.5 (manufactured by PRIMIX Corporation) at 1,500 rpm for 3 minutes. Thus, an emulsion 8 was prepared.

### [Comparative Example 3]

50 parts by mass (150 g) of the component (A) [2] amino-modified silicone (viscosity: 80,000 mPa·s, amine equivalent: 20,000 g/mol) (1a) shown by the formula (3), 1.0 parts by mass (3 g) of the component (B) [2] polyoxyethylene alkyl (having 12 carbon atoms) ether (4 EO) (LEOX CL-40, manufactured by Lion Corporation) (2b), 4.0 parts by mass (12 g) of the component (B) [3] polyoxyethylene alkyl (having 12 carbon atoms) ether (23 EO) (LEOX CL-230, manufactured by Lion Corporation) (2c), 0.5 parts by mass (1.5 g) of the component (F) [1] sodium polyoxyethylene lauryl ether sulfate (3 EO) (25% aqueous solution, EMAL 20C, manufactured by Kao Corporation), and 5.5 parts by mass (16.5 g) of the component (D) [1]-1 water were charged into HOMOGENIZING DISPER Model 2.5 (manufactured by PRIMIX Corporation) and stirred at 1,500 rpm for 40 minutes. Subsequently, 37.74 parts by mass (113.22 g) of the component (D) [1]-2 water was added, and the resultant was stirred with HOMOGENIZING MIXER MARK II Model 2.5 (manufactured by PRIMIX Corporation) at 1,500 rpm for 3 minutes for dilution. After that, 0.26 parts by mass (0.78 g) of an acid component [4]-2 phosphoric acid (85% aqueous solution, manufactured by Hayashi Pure Chemical Ind., Ltd.) (3d) was blended, and the resultant was stirred with HOMOGENIZING MIXER MARK II Model 2.5 (manufactured by PRIMIX Corporation) at 1,500 rpm for 3 minutes. Next, 1.0 parts by mass (3 g) of phenoxy ethanol was blended as an antiseptic, and the resulting mixture was stirred with HOMOGENIZING MIXER MARK II Model 2.5 (manufactured by PRIMIX Corporation) at 1,500 rpm for 3 minutes. Thus, an emulsion 9 was prepared.

### [Comparative Example 4]

50 parts by mass (150 g) of the component (A) [2] amino-modified silicone (viscosity: 80,000 mPa·s, amine equivalent: 20,000 g/mol) (1a) shown by the formula (3), 1.0 parts by mass (3 g) of the component (B) [2] polyoxyethylene alkyl (having 12 carbon atoms) ether (4 EO) (LEOX CL-40, manufactured by Lion Corporation) (2b), 4.0 parts by mass (12g) of the component (B) [3] polyoxyethylene alkyl (having 12 carbon atoms) ether (23 EO) (LEOX CL-230, manufactured by Lion Corporation) (2c), 0.5 parts by mass (1.5 g) of the component (F) [1] sodium polyoxyethylene lauryl ether sulfate (3 EO) (25% aqueous solution, EMAL 20C, manufactured by Kao Corporation), and 5.5 parts by mass (16.5 g) of the component (D) [1]-1 water were charged into HOMOGENIZING DISPER Model 2.5 (manufactured by PRIMIX Corporation) and stirred at 1,500 rpm for 40 minutes. Subsequently, 37.69 parts by mass (113.07 g) of the component (D) [1]-2 water was added, and the resultant was stirred with HOMOGENIZING MIXER MARK II Model 2.5 (manufactured by PRIMIX Corporation) at 1,500 rpm for 3 minutes for dilution. After that, 0.31 parts by mass (0.93 g) of an acid component [5]-2 salicylic acid (manufactured by Yoshitomi Pharmaceutical Industries, Ltd.) (3e) was blended, and the resultant was stirred with HOMOGENIZING MIXER MARK II Model 2.5 (manufactured by PRIMIX Corporation) at 1,500 rpm for 3 minutes. When 1.0 parts by mass (3 g) of phenoxy ethanol was subsequently blended as an antiseptic and the resulting mixture was stirred with HOMOGENIZING MIXER MARK II Model 2.5 (manufactured by PRIMIX Corporation) at 1,500 rpm for 3 minutes, a large amount of gel was generated.

### (Component (A))

Component (A) [1]: the amino-modified silicone shown by the formula (2)
   Viscosity: 800 mPa·s, amine equivalent: 1,500 g/mol
   Octamethyl cyclotetrasiloxane (D4): 100 ppm
   Decamethyl cyclopentasiloxane (D5): 150 ppm
   Dodecamethyl cyclohexasiloxane (D6): 500 ppm
Component (A) [2]: the amino-modified silicone shown by the formula (3)
   Viscosity: 80,000 mPa·s, amine equivalent: 20,000 g/mol
   Octamethyl cyclotetrasiloxane (D4): less than 100 ppm
   Decamethyl cyclopentasiloxane (D5): 200 ppm
   Dodecamethyl cyclohexasiloxane (D6): 700 ppm
Component (A) [3]: the amino-modified silicone shown by the formula (4)
   Viscosity: 1,800 mPa·s, amine equivalent: 3,500 g/mol
   Octamethyl cyclotetrasiloxane (D4): 1,500 ppm
   Decamethyl cyclopentasiloxane (D5): 2,000 ppm
   Dodecamethyl cyclohexasiloxane (D6): 2,000 ppm

### (Component (B))

Component (B) [1]: SANNONIC SS-120 (manufactured by Sanyo Chemical Industries, Ltd.) (2a)
   Polyoxyethylene alkyl (having 12 to 14 carbon atoms) ether (12 EO)
Component (B) [2]: LEOX CL-40 (manufactured by Lion Corporation) (2b)
   Polyoxyethylene alkyl (having 12 carbon atoms) ether (4 EO)
Component (B) [3]: LEOX CL-230 (manufactured by Lion Corporation) (2c)
   Polyoxyethylene alkyl (having 12 carbon atoms) ether (23 EO)

### (Component (C))

Component (C) [1]: lactic acid (90% aqueous solution, manufactured by Musashino Chemical Laboratory, Ltd.) (3a)
[2]: acetic acid (30% aqueous solution, manufactured by Kozakai Pharmaceutical Co., Ltd.) (3b) Acid component [3]: propionic acid (manufactured by Kanto Chemical Co., Inc.) (3c)
[4]: phosphoric acid (manufactured by Hayashi Pure Chemical Ind., Ltd.) (3d)
[5]: salicylic acid (manufactured by Yoshitomi Pharmaceutical Industries, Ltd.) (3e)

### (Component (E))

Component (E) [1]: 1,3-butylene glycol P (manufactured by KH Neochem Co., Ltd.) (4a)

### (Component (F))

Component (F) [1]: EMAL 20C (25% aqueous solution, manufactured by Kao Corporation) (5a)
Sodium polyoxyethylene lauryl ether sulfate (3 EO)

Average particle size, pH, viscosity, and increased amount of cyclic low-molecular-weight siloxanes were measured regarding the Examples and the Comparative Examples. The results are shown in Tables 1 to 3.

Average particle size:
the value was obtained by diluting the emulsified compositions (emulsions) 1 to 9 to about 10 times with water and measuring using particle size measuring equipment (LA960 manufactured by HORIBA, Ltd.) in the case of the emulsified compositions 1 to 5 and 7 to 9, and using particle size measuring equipment (N4 PLUS manufactured by BECKMAN COULTER) in the case of emulsified composition 6.

pH:
the value was obtained by measuring the emulsified compositions 1 to 9 at 25°C with a pH meter (LAQUA manufactured by HORIBA, Ltd.).

Viscosity:
the value was obtained by measuring the emulsified compositions 1 to 9 at 25°C with a rotational viscometer (VISCOMETER TVB-10 manufactured by Toki Sangyo Co., Ltd.) at a rotational rate of 30 rpm.

Increased amount of cyclic low-molecular-weight siloxanes:
the increased amount of cyclic low-molecular-weight siloxanes (octamethyl cyclotetrasiloxane (D4), decamethyl cyclopentasiloxane (D5), and dodecamethyl cyclohexasiloxane (D6)) in each of the emulsified compositions relative to the component (A) when the emulsified compositions 1 to 9 had been stored at 25°C for 6 months (the increased amount of the cyclic low-molecular-weight siloxanes relative to the component (A) after storing for 6 months from immediately after production).

The methods for measuring the cyclic low-molecular-weight siloxanes were as follows.

### [Component (A) Amino-Modified Silicone and Emulsified Compositions 4 and 7 to 9]

A small amount of 0.1 g of a measurement sample was added to 10 mL of acetone and shaken for about 2 hours. After extracting the cyclic low-molecular-weight siloxanes into the acetone solution by the shaking, supernatant acetone solution was measured by gas chromatography (Agilent 7890B (manufactured by Agilent Technologies Japan, Ltd.)).

### [Component (A) Amino-Modified Silicone and Emulsified Compositions 1 to 3, 5, and 6]

A small amount of 0.1 g of a measurement sample and 0.1 g of dehydrated mirabilite were added to 10 mL of hexane and shaken for about 2 hours. After extracting the cyclic low-molecular-weight siloxane into the hexane solution by the shaking, supernatant hexane solution was measured by gas chromatography (Agilent 7890B (manufactured by Agilent Technologies Japan, Ltd.)).

**[Table 1]**

| Composition (%) | | | Example | | | | |
|---|---|---|---|---|---|---|---|
| | | | 1 | 2 | 3 | 4 | 5 |
| Component (A) | [1] | | 30 | 30 | 30 | | 30 |
| | [2] | | | | | 50 | |
| Component (B) | [1] | | 5.0 | 5.0 | 5.0 | | 5.0 |
| | [2] | | | | | 1.0 | |
| | [3] | | | | | 4.0 | |
| Component (C) | [1] -1 | | | | | | 0.38 |
| | [1] -2 | | 1.8 | 1.3 | | | 0.92 |
| | [2]-2 | | | | 2.6 | 0.45 | |
| Component (D) | [1] -1 | | 13.5 | 13.5 | 13.5 | 5.5 | 12 |
| | [1] -2 | | 46.7 | 47.2 | 45.9 | 37.55 | 46.7 |
| Component (E) | [1] | | 2.0 | 2.0 | 2.0 | | 4.0 |
| Component (F) | [1] | | | | | 0.5 | |
| Phenoxy ethanol | | | 1.0 | 1.0 | 1.0 | 1.0 | 1.0 |
| Total | | | 100 | 100 | 100 | 100 | 100 |
| Proportion of component (C) (mol%) relative to amino groups | | | 90% | 65% | 65% | 90% | 65% |
| Emulsified composition (emulsion) | | | 1 | 2 | 3 | 4 | 5 |
| Average particle size (nm) | | | 170 | 180 | 300 | 400 | 170 |
| pH | | | 5. 6 | 6.3 | 5. 9 | 4.5 | 5.6 |
| Viscosity (mPa·s) | | | 2, 600 | 160 | 890 | 800 | 1, 100 |
| Increased amount of cyclic low-molecular-weight siloxanes (ppm) | | D4 | 300 | 1200 | 2,300 | Less than 100 | 400 |
| | | D5 | Less than 100 | Less than 100 | 200 | Less than 100 | Less than 100 |
| | | D6 | Less than 100 | Less than 100 | Less than 100 | Less than 100 | Less than 100 |

**[Table 2]**

| Composition (%) | | | Example |
|---|---|---|---|
| | | | 6 |
| Component (A) | [1] | | |
| | [3] | | 30 |
| Component (B) | [1] | | 10 |
| | [2] | | |
| | [3] | | |
| Component (C) | [1] -1 | | 0.6 |
| | [1] -2 | | |
| | [2] -2 | | |
| Component (D) | [1] -1 | | 12 |
| | [1] -2 | | 41.9 |
| Component (E) | [1] | | 4.0 |
| Component (F) | [1] | | 0.5 |
| Phenoxy ethanol | | | 1.0 |
| Total | | | 100 |
| Proportion of component (C) (mol%) relative to amino groups | | | 70% |
| Emulsified composition (emulsion) | | | 6 |
| Average particle size (nm) | | | 50 |
| pH | | | 6.5 |
| Viscosity mPa·s | | | 200 |
| Increased amount of cyclic low-molecular-weight siloxanes (ppm) | | D4 | 1,200 |
| | | D5 | 300 |
| | | D6 | Less than 100 |

**[Table 3]**

| Composition (%) | | | Comparative Example | | | |
|---|---|---|---|---|---|---|
| | | | 1 | 2 | 3 | 4 |
| Component (A) | [1] | | 30 | 30 | | |
| | [2] | | | | 50 | 50 |
| Component (B) | [1] | | 5.0 | 5.0 | | |
| | [2] | | | | 1.0 | 1.0 |
| | [3] | | | | 4.0 | 4.0 |
| Component (C) and acid component | [1] -1 | | | | | |
| | [1] -2 | | 0.6 | | | |
| | [3] -2 | | | 0.96 | | |
| | [4]-2 | | | | 0.26 | |
| | [5] -2 | | | | | 0.31 |
| Component (D) | [1] -1 | | 13.5 | 13.5 | 5.5 | 5.5 |
| | [1] -2 | | 47.9 | 47.54 | 37.74 | 37.69 |
| Component (E) | [1] | | 2.0 | 2.0 | | |
| Component (F) | [1] | | | | 0.5 | 0.5 |
| Phenoxy ethanol | | | 1.0 | 1.0 | 1.0 | 1.0 |
| Total | | | 100 | 100 | 100 | 100 |
| Proportion of component (C) (mol%) relative to amino groups | | | 30% | 65% | 90% | 90% |
| Emulsified composition (emulsion) | | | 7 | 8 | 9 | - |
| Average particle size (nm) | | | 280 | 250 | 450 | - |
| pH | | | 7.8 | 6.1 | 2.7 | - |
| Viscosity mPa·s | | | 20 | 130 | 180 | - |
| Increased amount of cyclic low-molecular-weight siloxanes (ppm) | | D4 | 3, 000 | 3,000 | 3,000 | - |
| | | D5 | 100 | 500 | Less than 100 | - |
| | | D6 | Less than 100 | Less than 100 | Less than 100 | - |

From the results of Tables 1 to 3, it is obvious that each of octamethyl cyclotetrasiloxane (D4), decamethyl cyclopentasiloxane (D5), and dodecamethyl cyclohexasiloxane (D6) hardly increase over time in the inventive amino-modified silicone emulsions compared with the amino-modified silicone emulsions of Comparative Examples 1 to 4.

That is, in Comparative Example 1, in which the blended amount of the component (C) was less than the range of the present invention, the pH of the emulsion was higher than the pH of the Examples, and in particular, the amount of (D4) after storage at 25°C for 6 months had greatly increased.

The amount of (D4) also greatly increased in Comparative Examples 2 and 3, in which propionic acid or phosphoric acid was used instead of lactic acid or acetic acid, which are the component (C) of the present invention.

In Comparative Example 4, in which salicylic acid was used, a large amount of gel was generated, so that it was not possible to obtain an emulsion in the first place.

### [Hair Cosmetic]

The composition of Example 1 was blended in a hair treatment having the following composition (pH 4.0) to prepare a hair cosmetic in the usual manner

| composition | % |
|---|---|
| dimethyl behenamine | 2.0 |
| behenyltrimethylammonium chloride | 0.3 |
| stearyl alcohol | 4.5 |
| behenyl alcohol | 1.5 |
| isononyl isononanoate | 0.5 |
| highly polymerized dimethylpolysiloxane*1 | 1.0 |
| Example 1 | 1.5 |
| glycolic acid | 0.5 |
| malic acid | 1.5 |
| dipropylene glycol | 0.1 3.0 |
| benzyl alcohol | 0.3 |
| arginine | 0.2 |
| pantothenyl ethyl ether | 0.1 |
| hydrolyzed conchiolin liquid (dry portion 3%) | 0.05 |
| panax ginseng extract (dry portion 3%) | 0.05 |
| soybean extract (dry portion 0.4%) | 0.05 |
| eucalyptus extract (dry portion 0.2%) | 0.05 |
| rice germ oil | 0.05 |
| fragrance | appropriate amount |
| methyl paraben | appropriate amount |
| purified water | remainder |
| total | 100.0 |

| | |
|---|---|
| *1 KF-96H 100000 cs (Shin-Etsu Chemical Co., Ltd.) | |

The obtained hair treatment gave favorable results of smoothness, softness, and flatness in rinsing; and favorable results of smoothness, softness, and easy combing after drying.

### [Fiber Treatment Agent (Softener)]

Example 6 was diluted to 1% with ion-exchanged water, and applied to a pair of commercially available facial tissues ("Nepia funwari surimu": manufactured by Oji Nepia, Co., Ltd., length 197 mm × width 229 mm, two-layer, mass: 1.0 g) in five places from 15 cm away by spraying, and air-dried at room temperature for 6 hours.

The obtained facial tissues were excellent in both smoothness and softness.

Thus, it has been revealed that in the inventive amino-modified silicone emulsion, octamethyl cyclotetrasiloxane (D4), decamethyl cyclopentasiloxane (D5), and dodecamethyl cyclohexasiloxane (D6) hardly increase over time compared with conventional products, and that when the inventive amino-modified silicone emulsion is blended in a hair cosmetic or a fiber treatment agent, excellent smoothness and softness can be provided.

It should be noted that the present invention is not limited to the above-described embodiments. The embodiments are just examples, and any examples that have substantially the same feature and demonstrate the same functions and effects as those in the technical concept disclosed in claims of the present invention are included in the technical scope of the present invention.

## Claims

1. An amino-modified silicone emulsion comprising:
(A) an amino-modified silicone that is represented by the following general formula (1), has a viscosity at 25°C of 200 to 100,000 mPa·s, and has an amine equivalent of 1,000 to 20,000 g/mol, 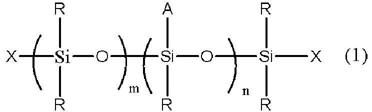 wherein R represents a substituted or unsubstituted monovalent hydrocarbon group having 1 to 12 carbon atoms, A represents a monovalent group shown by - R¹(NR²CH₂CH₂)ₐNR³R⁴, wherein R¹ represents a divalent hydrocarbon group having 1 to 8 carbon atoms, R², R³, and R⁴ each independently represent a hydrogen atom or a monovalent hydrocarbon group having 1 to 4 carbon atoms, and "a" represents an integer of 0 to 4, X represents a monovalent group shown by R, A, or -OR⁵, R⁵ represents a hydrogen atom or a monovalent hydrocarbon group having 1 to 8 carbon atoms, and "m" and "n" are mean values and are numbers that satisfy the viscosity and the amine equivalent, provided that when n=0, at least one X is A, and that octamethyl cyclotetrasiloxane (D4), decamethyl cyclopentasiloxane (D5), and dodecamethyl cyclohexasiloxane (D6) are each contained in an amount of less than 3,000 ppm relative to a mass of the component (A);
(B) 5 to 100 mass% of a nonionic surfactant relative to the mass of the component (A);
(C) 50 to 100 mol% of lactic acid or acetic acid relative to a number of moles of amino groups of the component (A); and
(D) 85 to 10,000 mass% of water relative to the mass of the component (A),
wherein an increased amount of each of (D4), (D5), and (D6) is less than 3,000 ppm relative to the mass of the component (A) when an emulsion of the amino-modified silicone has been stored at 25°C for 6 months.

2. The amino-modified silicone emulsion according to claim 1, comprising (E) 1 to 20 mass% of a polyhydric alcohol relative to the mass of the component (A).

3. The amino-modified silicone emulsion according to claim 1 or 2, comprising (F) 0.1 to 3 mass% of an anionic surfactant relative to the mass of the component (A).

4. The amino-modified silicone emulsion according to any one of claims 1 to 3, wherein the nonionic surfactant (B) is a polyoxyalkylene alkyl ether.

5. The amino-modified silicone emulsion according to any one of claims 1 to 4, wherein the increased amount of each of (D4), (D5), and (D6) is less than 2,000 ppm relative to the mass of the component (A) when stored at 25°C for 6 months.

6. A hair cosmetic comprising 0.1 to 20 mass% of the amino-modified silicone emulsion according to any one of claims 1 to 5.

7. A fiber treatment agent comprising 0.1 to 20 mass% of the amino-modified silicone emulsion according to any one of claims 1 to 5.

8. The amino-modified silicone emulsion according to any one of claims 1 to 5, wherein in the component (A), octamethyl cyclotetrasiloxane (D4), decamethyl cyclopentasiloxane (D5), and dodecamethyl cyclohexasiloxane (D6) are each contained in an amount of less than 2,000 ppm relative to a mass of the component (A).

9. The amino-modified silicone emulsion according to any one of claims 1 to 5, wherein in the component (A), octamethyl cyclotetrasiloxane (D4), decamethyl cyclopentasiloxane (D5), and dodecamethyl cyclohexasiloxane (D6) are each contained in an amount of less than 1,000 ppm relative to a mass of the component (A).

## Patentansprüche

1. Aminomodifizierte Siliconemulsion, die umfasst:
(A) ein aminomodifiziertes Silicon, das durch die folgende allgemeine Formel (1) repräsentiert wird, eine Viskosität bei 25°C von 200 bis 100.000 mPa·s hat und ein Aminäquivalent von 1.000 bis 20.000 g/mol aufweist, 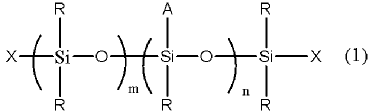 wobei R eine substituierte oder unsubstituierte einwertige Kohlenwasserstoffgruppe mit 1 bis 12 Kohlenstoffatomen repräsentiert, A eine einwertige Gruppe repräsentiert, die durch
-R¹(NR²CH₂CH₂)ₐNR³R⁴ dargestellt wird, wobei R¹ eine zweiwertige Kohlenwasserstoffgruppe mit 1 bis 8 Kohlenstoffatomen repräsentiert, R², R³ und R⁴ jeweils unabhängig voneinander ein Wasserstoffatom oder eine einwertige Kohlenwasserstoffgruppe mit 1 bis 4 Kohlenstoffatomen repräsentieren, und "a" eine ganze Zahl von 0 bis 4 repräsentiert, X eine einwertige Gruppe repräsentiert, die durch R, A oder -OR⁵ dargestellt wird, R⁵ ein Wasserstoffatom oder eine einwertige Kohlenwasserstoffgruppe mit 1 bis 8 Kohlenstoffatomen repräsentiert und "m" und "n" Mittelwerte sind und Zahlen sind, die die Viskosität und das Aminäquivalent erfüllen, mit der Maßgabe, dass, wenn n=0, mindestens ein X A ist und dass Octamethylcyclotetrasiloxan (D4), Decamethylcyclopentasiloxan (D5) und Dodecamethylcyclohexasiloxan (D6) jeweils in einer Menge von weniger als 3.000 ppm, bezogen auf eine Masse der Komponente (A), enthalten sind;
(B) 5 bis 100 Masse-% eines nichtionischen Oberflächenbehandungsmittels, bezogen auf die Masse der Komponente (A);
(C) 50 bis 100 Mol-% Milchsäure oder Essigsäure, bezogen auf eine Molzahl von Aminogruppen der Komponente (A); und
(D) 85 bis 10.000 Masse-% Wasser, bezogen auf die Masse der Komponente (A),
wobei eine erhöhte Menge von (D4), (D5) und (D6) jeweils weniger als 3.000 ppm, bezogen auf die Masse der Komponente (A), beträgt, wenn eine Emulsion des aminomodifizierten Silicons 6 Monate lang bei 25°C gelagert wurde.

2. Aminomodifizierte Siliconemulsion nach Anspruch 1, die (E) 1 bis 20 Masse-% eines mehrwertigen Alkohols, bezogen auf die Masse der Komponente (A), umfasst.

3. Aminomodifizierte Siliconemulsion nach Anspruch 1 oder 2, die (F) 0,1 bis 3 Masse-% eines anionischen Oberflächenbehandungsmittels, bezogen auf die Masse der Komponente (A), umfasst.

4. Aminomodifizierte Siliconemulsion nach einem der Ansprüche 1 bis 3, wobei das nichtionische Oberflächenbehandungsmittel (B) ein Polyoxyalkylenalkylether ist.

5. Aminomodifizierte Siliconemulsion nach einem der Ansprüche 1 bis 4, wobei die erhöhte Menge von (D4), (D5) und (D6) jeweils weniger als 2.000 ppm, bezogen auf die Masse der Komponente (A), beträgt, wenn sie 6 Monate lang bei 25°C gelagert wird.

6. Haarkosmetikum, das 0,1 bis 20 Masse-% der aminomodifizierten Silikonemulsion nach einem der Ansprüche 1 bis 5 umfasst.

7. Faserbehandlungsmittel, das 0,1 bis 20 Masse-% der aminomodifizierten Silikonemulsion nach einem der Ansprüche 1 bis 5 umfasst.

8. Aminomodifizierte Siliconemulsion nach einem der Ansprüche 1 bis 5, wobei in der Komponente (A) Octamethylcyclotetrasiloxan (D4), Decamethylcyclopentasiloxan (D5) und Dodecamethylcyclohexasiloxan (D6) jeweils in einer Menge von weniger als 2.000 ppm, bezogen auf eine Masse der Komponente (A), enthalten sind.

9. Aminomodifizierte Siliconemulsion nach einem der Ansprüche 1 bis 5, wobei in der Komponente (A) Octamethylcyclotetrasiloxan (D4), Decamethylcyclopentasiloxan (D5) und Dodecamethylcyclohexasiloxan (D6) jeweils in einer Menge von weniger als 1.000 ppm, bezogen auf die Masse der Komponente (A), enthalten sind.

## Revendications

1. Émulsion de silicone amino-modifiée comprenant:
(A) une silicone amino-modifiée qui est représentée par la formule générale suivante (1), dont la viscosité à 25°C s'élève de 200 à 100 000 mPa·s, et dont l'équivalent d'amine est de 1 000 à 20 000 g/mol, 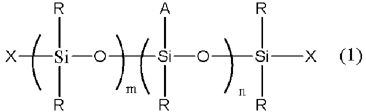 dans laquelle R représente un groupe hydrocarbure monovalent substitué ou non substitué ayant 1 à 12 atomes de carbone, A représente un groupe monovalent représenté par -R¹(NR²CH₂CH₂)ₐNR³R⁴, dans lequel R¹ représente un groupe hydrocarbure divalent ayant 1 à 8 atomes de carbone, R², R³, et R⁴ représentent chacun indépendamment un atome d'hydrogène ou un groupe hydrocarbure monovalent ayant 1 à 4 atomes de carbone, et « a » représente un nombre entier de 0 à 4, X représente un groupe monovalent représenté par R, A, - OR⁵, R⁵ représentant un atome d'hydrogène ou un groupe hydrocarbure monovalent ayant de 1 à 8 atomes de carbone, et « m » et « n » étant des valeurs moyennes et des nombres qui satisfont à la viscosité et à l'équivalent amine, à condition que lorsque n=0, au moins un X ne soit A, et que l'octaméthylcyclotétrasiloxane (D4), le décaméthylcyclopentasiloxane (D5) et le dodécaméthylcyclohexasiloxane (D6) soient contenus chacun en une quantité inférieure à 3 000 ppm par rapport à la masse du constituant (A);
(B) 5 à 100 % en masse d'un agent de surface non ionique par rapport à la masse du constituant (A);
(C) 50 à 100 % en moles d'acide lactique ou d'acide acétique par rapport au nombre de moles de groupes amine du constituant (A); et
(D) 85 à 10 000 % en masse d'eau par rapport à la masse du constituant (A),
dans lequel une quantité accrue de chacun parmi (D4), (D5) et (D6) est inférieure à 3 000 ppm par rapport à la masse du constituant (A) lorsqu'une émulsion de silicone amino-modifiée a été stockée à 25°C pendant 6 mois.

2. Émulsion de silicone amino-modifiée selon la revendication 1, comprenant (E) 1 à 20 % en masse d'un alcool polyhydrique par rapport à la masse du constituant (A).

3. Émulsion de silicone amino-modifiée selon la revendication 1 ou 2, comprenant (F) 0,1 à 3 % en masse d'un agent de surface anionique par rapport à la masse du constituant (A).

4. Émulsion de silicone amino-modifiée selon l'une quelconque des revendications 1 à 3, dans laquelle l'agent de surface non ionique (B) est un éther alkyl polyoxyalkylène.

5. Silicone amino-modifiée selon l'une quelconque des revendications 1 à 4, dans laquelle la quantité accrue de chacun parmi (D4), (D5) et (D6) est inférieure à 2 000 ppm par rapport à la masse du constituant (A) lorsqu'il est stocké à 25°C pendant 6 mois.

6. Produit capillaire comprenant de 0,1 à 20 % en masse de l'émulsion de silicone amino-modifiée selon l'une quelconque des revendications 1 à 5.

7. Agent de traitement des fibres comprenant de 0,1 à 20 % en masse de l'émulsion de silicone amino-modifiée selon l'une quelconque des revendications 1 à 5.

8. Émulsion de silicone amino-modifiée selon l'une quelconque des revendications 1 à 5, dans laquelle dans le constituant (A), l'octaméthylcyclotétrasiloxane (D4), le décaméthylcyclopentasiloxane (D5) et le dodécaméthylcyclohexasiloxane (D6) sont contenus chacun en une quantité inférieure à 2 000 ppm par rapport à la masse du constituant (A).

9. Émulsion de silicone amino-modifiée selon l'une quelconque des revendications 1 à 5, dans laquelle dans le constituant (A), l'octaméthylcyclotétrasiloxane (D4), le décaméthylcyclopentasiloxane (D5) et le dodécaméthylcyclohexasiloxane (D6) sont contenus chacun en une quantité inférieure à 1 000 ppm par rapport à la masse du constituant (A).
